# NEW EUROPEAN PATENT SPECIFICATION

(11) **EP 1 044 283 B2**
(45) Date of publication and mention of the opposition decision: **23.09.2015**
(45) Mention of the grant of the patent: 10.05.2006
(21) Application number: 98955810.1
(22) Date of filing: 27.11.1998
(51) Int. Cl.: C12Q 1/68

(54) **FLUOROMETRIC METHOD FOR MONITORING AND DETECTING NUCLEIC ACID AMPLIFICATION**
FLUOROMETRISCHES VERFAHREN ZUR ÜBERWACHUNG UND ZUM NACHWEIS VON NUCLEINSÄURE-AMPLIFIZIERUNG
SYSTEME FLUOROMETRIQUE D'OBSERVATION ET DE DETECTION DE L'AMPLIFICATION D'ACIDES NUCLEIQUES

(30) Priority: 29.11.1997 GB 9725237
(43) Date of publication of application: 18.10.2000
(60) Divisional application: 06005863.3
(73) Proprietor: THE SECRETARY OF STATE FOR DEFENCE, Salisbury, Wiltshire SP4 0JQ (GB)
(72) Inventor: LEE, Martin, Alan, Salisbury Wiltshire SP4 0JG (GB); LESLIE, Dario, Lyall, Salisbury Wiltshire SP4 0JG (GB)
(74) Representative: Beckham, Robert William
(86) International application number: PCT/GB1998/003563
(87) International publication number: WO 1999/028501

(56) References cited:
- EP-A1- 0 684 315
- EP-A2- 0 878 554
- WO-A-96/21144
- WO-A-97/41256
- DE-A- 4 419 566
- US-A- 5 763 181
- S. GHOSH ET AL.: "Real time kinetics of restriction endonuclease cleavage monitored by fluorescence resonance energy transfer" NUCLEIC ACIDS RESEARCH, vol. 22, no. 15, 1994, pages 3155-3159, XP002054293 GB
- K.J. LIVAK ET AL.: "Oligonucleotides with fluorescent dyes at opposite ends provide a quenched probe system useful for detecting PCR product and nucleic acid hybridisation" PCR METHODS AND APPLICATIONS, vol. 4, June 1995, pages 357-362, XP000522969 US cited in the application
- NAZARENKO I.A. ET AL.: "A closed tube format for amplification and detection of DNA based on energy transfer" NUCLEIC ACIDS RESEARCH, vol. 25, no. 12, 1997, pages 2516-2521, XP002094959 GB cited in the application
- D. H. PERSING ET AL.: 'Diagnostic Molecular Microbiology', 1994, AMERICAN SOCIETY FOR MICROBIOLOGY, WASHINGTON pages 65 - 67
- A. JELTSCH ET AL.: 'A Fast and Accurate Enzyme-Linked Immunosorbent Assay for the Determination of the DNA Cleavage Activity of Restriction Endonucleases' ANALYTICAL BIOCHEMISTRY vol. 213, 1993, pages 234 - 240

## Description

The present invention provides a method for detecting amplification of a target polynucleotide (sometimes known as a poly nucleic acid), preferably quantitatively, and to probes for use in those methods.

Known fluorescence PCR monitoring techniques include both strand specific and generic DNA intercalator techniques that can be used on a few second-generation PCR thermal cycling devices.

Generic methods utilise DNA intercalating dyes that exhibit increased fluorescence when bound to double stranded DNA species. Fluorescence increase due to a rise in the bulk concentration of DNA during amplifications can be used to measure reaction progress and to determine the target molecule copy number. However, these methods are only quasi strand-specific since any non-specific amplification which takes place will generate double stranded DNA and so produce an increase in signal.

Strand specific methods utilise additional polynucleotide reaction components to monitor the progress of amplification reactions. These methods use fluorescence resonance transfer (FRET) as the basis of detection. One or more polynucleotide probes are labelled with fluorescent molecules, a donor molecule and an acceptor molecule (sometimes known as a reporter and a quencher molecule respectively). The donor molecule is excited with a specific wavelength of light for which it will normally exhibit a fluorescence emission wavelength. The acceptor molecule is highly excited at the emission wavelength such that it can accept the emission energy of the donor molecule by resonance transfer when they are in close proximity (e.g. on the same, or a neighbouring molecule). The basis of FRET detection is to monitor the changes at donor and acceptor emission wavelengths. There are two types of FRET probes, those using hydrolysis of polynucleotide probes to separate donor from acceptor, and those using hybridisation to alter the spatial relationship of donor and acceptor molecules.

Hydrolysis probes are commericially available as TaqMan™ probes. These consist of DNA oligonucloetides that are labelled with donor and acceptor molecules. The probes are designed to bind to a specific region on one strand of a PCR product. Following annealing of the PCR primer to this strand, *Taq* enzyme extends the DNA with 5' to 3' polymerase activity. *Taq* enzyme also exhibites 5' to 3' exonuclease activity. TaqMan^{™} probes are protected at the 3' end by phosphorylation to prevent them from priming *Taq* extension. If the TaqMan^{™} probe is hybridised to the product strand than an extending *Taq* molecule may also hydrolyse the probe, liberating the donor from acceptor as the basis of detection.

Hybridisation probes are available in a number of guises. Molecular beacons are oligonucleotides that have complementary 5' and 3' sequences such that they form hairpin loops. Terminal fluorescent labels are in close proximity for FRET to occur when the hairpin structure is formed. Following hybridisation of molecular beacons to a complementary sequence the fluorescent labels are separated, so FRET does not occur, as the basis of detection. A modification of such a system is in which the molecular beacon is attached to an amplification primer is described in Nucl. Acids. Res. (1997) 25, 12, 2516-2521.

However, the signal generated by such sequences is limited by the length of the probe as this forms the limiting factor in the level of signal to noise ratio which can be achieved.

WO-A-96211446 discloses a method for the detection of an amplification reaction comprising the user of a probe fluorescently labelled at both ends and of a restriction enzyme.

The applicants have developed a system which allows accurate detection and/or monitoring of certain reactions including amplification reactions.

Thus the invention provides a method for detecting nucleic acid amplification comprising performing nucleic acid amplification on a target polynucleotide in the presence of (a) a nucleic acid polymerase, (b) a primer capable of hybridising to said target polynucleotide, and (c) an oligonucleotide probe which is capable of hybridising to said target polynucleotide and which comprises a fluorescence donor molecule, a fluorescence acceptor molecule and a single stranded sequence recognised by a restriction enzyme which cuts at said sequence when in double stranded form, said sequence being located intermediate said donor and said acceptor molecule; applying to the amplification product said restriction enzyme such that double stranded amplification product is cleaved so as to liberate acceptor molecule from donor molecule molecule, and detecting a fluorescent signal from the reaction mixture, wherein the probe is designed so that the oligonucleotide probe is capable of hybridising to said target polynucleotide in the region of the 3' end of said polynucleotide that can extend in the presence of the polymerase to provide the complementary strand double stranded DNA with the oligonucleotide probe. The restriction site is suitably located in the extension region. In this case, the target polynucleotide will itself act as a primer for extension and during a chain extension reaction, the polynucleotide sequence will extend along the length of the probe thereby rendering it double stranded. Once this occurs, the restriction enzyme can cut the extended region, thereby releasing the acceptor from the probe so as to generate a signal.

Suitably the 3' end of the probe is 'blocked' for example by phosphorylation so that it does not extend in the direction of the polynucleotide during extension phases of the amplification cycle.

This means that the polymerase extension reaction will result in extension of the target polynucleotide strand in the region complementary to the overhanging region of the probe, resulting in the production of a double stranded restriction site in this region. Where the enzyme is selected such that the restriction site is not found elsewhere in the amplicon, application of the restriction enzyme will not cut within the target polynucleotide sequence and so full length product may afterwards be isolated.

The method can be used to detect the presence of a target polynucleotide in sample. If amplification is detected in the method of the invention, then target polynucleotide is present in the sample to act as a template.

By using a restriction enzyme to liberate donor molecule from acceptor molecule, the time delay incurred for example when multiple hydrolysis reactions are required in order to bring about the separation is avoided.

Furthermore, the separation of the acceptor from the donor molecule is not as limited by the length of the probe and therefore a better signal to noise ratio may be achieved.

The method of the invention may be used for end point detection, where the enzyme is added after the amplification reaction, or the enzyme may be present throughout the amplification reaction. The latter allows the potential for monitoring the amplification reaction in real time. However, in some cases, the reagents required for the enzyme to digestion may inhibit the amplification reaction. In these cases, end point detection in a separate tube may be desirable.

The oligonucleotide probe may be designed such that it includes the site for the restriction enzyme within the region of the amplicon. In this case, detection of amplification is effected by adding the restriction enzyme to the reaction on completion. The enzyme cuts the amplification product between the donor and acceptor molecule of the probe, thus liberating the donor molecule which generates an end-point signal. This may be used for in-situ PCR situations. Its use however depends upon the presence within the amplicon of a site which will hybridise with the probe. In addition, total isolation of full length amplicon may not be possible following detection in this manner.

Alternatively, the probe may be attached to an amplification primer. In such cases both the acceptor and donor molecules are preferably present on a non-binding region of the primer. When the thus produced amplicon undergoes a subsequent extension cycle under the influence of the other primer, the extension reaction will include the entire region of the probe thus making the restriction site double stranded and thereby liable to be cut by the restriction enzyme.

In addition, if the restriction enzyme is present throughout the amplification reaction, the progress of the amplification reaction itself may he monitored since donor molecules will be liberated in amounts which are proportional to the number of amplicons present in the reaction mixture at every cycle of the amplification reaction. The build up in the signal from the donor molecules may be observed and the increase used for example to calculate the amount of target polynucleotide present in the sample at the beginning using the sort of calculation which are well known in connection with the TaqMan^{™} system.

Restriction endonucleases are enzymes that bind to and cleave double stranded DNA molecules at specific base pair sequences known as restriction or recognition sites. There are three types of restriction enzymes. Each type has two specific enzyme activities. A DNA methylase and an endonuclease that catalyses the cleavage and separation of double stranded DNA.

Type I restriction enzymes have methylase and ATP dependent nuclease activities in the same molecule. Methylation occurs in the recognition site and cleavage occurs a distance away from the recognition site as the DNA loops around the bound enzyme. Type I and III enzymes are similar. Type III enzymes also have methylase and nuclease activities in the same molecule. However, the enzymes do not require ATP and the cleavage site is closer to the recognition site. Type II restriction enzymes cleave at the recognition site. They recognise structures four or more nucleotides in length. Each enzyme type cleaves differently. The enzyme can cleave at both strands in exactly the same place forming blunt ends on the DNA. Other enzymes can cleave at symmetrical positions on each strand of DNA producing fragments with single stranded overhangs or "sticky ends". They can create fragments with 3' overhangs and 5'overhangs.

Any of the known restriction enzymes may be used in the method of the invention, particularly where these are added at the end of any amplification reaction, provided only that where a specific reaction product is to be recovered, these do not contain internal sites recognised by the enzymes. Restriction enzymes which can withstand the conditions employed in the reaction, for example in the amplification reaction are thermostable enzymes.

A particularly suitable restriction enzyme for use in the method of the invention is Taql. This restriction enzyme is, like TAQ polymerase, obtainable from Thermus aquaticus. It will therefore withstand the conditions found during cycling carried out in amplification reactions such as the polymerase chain reaction (PCR). A further such enzyme is the thermostable restriction enzyme "PspG 1", (New England Biolabs). This enzyme is isolated from Pyrococcus sp and recognises CCWGG cutting in front of first C. Stability is 2 hours at 95°C and it will survive 30 cycles of block PCR.

The PCR reaction is a preferred amplification method in the context of the invention.

Suitably the amplicon and/or restriction enzyme are selected so that the site recognised by the enzyme does not appear in the amplicon. If necessary, available restriction enzymes can be isolated, mutated or engineered so that they are thermostable at the reaction temperatures required.

Suitably the nucleic acid polymerase used in the method of the invention is a thermostable polymerase such as TAQ polymerase.

The probe used in the method of the invention may take the form of a molecular beacon so that it forms a hairpin shape prior to hybridisation to the target polynucleotide. In this case, the probe has complementary sequences in the 5' region and the 3' region such that prior to binding to the target polynucleotide sequence, the 5' region and 3' region bind together. The restriction will in this case be located in a non-complementary region of the probe so that is remains single stranded before the probe hybridises to the target sequence.

Suitably the donor molecule is arranged at one end of the probe and the acceptor is at the other end. Thus the donor molecule may be arranged at the 5' end of the probe and the acceptor is at the 3' end or vice-versa. In such cases, the hairpin arrangement of the molecular beacon is particularly effective as the donor and acceptor are held in close proximity prior to hybridization to the target sequence.
Hence the risk of an unwanted background signal from the donor molecule is reduced.

On hybridisation of the probe to the target sequence, the hairpin is opened out, thereby spatially separating the acceptor from the donor molecule and so generating a signal. However, in the case of the invention, the restriction enzyme will cut the probe-target sequence hybrid intermediate the donor and acceptor molecules thus further separating these moieties and so enhancing the signal.

In yet a further modification of this, the probe is attached at one end thereof to an amplification primer. The probe may or may not be in the form of a hairpin. However, the region containing the donor and acceptor molecules should be arranged upstream of the 3' end of the primer. Extension of the primer leads to an amplicon having the probe attached at the 5' end thereof. During a subsequent amplification cycle, a complementary strand is formed following the binding of the other amplification primer to the remote end of the amplicon. This complementary strand will be extended during chain extension, not only along the length of the target chain, but also across the probe, thereby rendering it double stranded and so liable for cutting by the restriction enzyme.

In yet a further embodiment of the invention, the amplification reaction is effected using modified nucleotides which are resistant to cutting by restriction enzymes. Examples of such nucleotides are methylated nucleotides. In this case, the probe used in the process will not contain modified nucleotides. This means that the amplicons produced in the reaction will be restriction resistant.

However when the probe hybridises to an amplicon, it will be recognised by the restriction enzyme which would then cut it at the restriction site. The complementary strand will not be cut however and so the result is that the double stranded DNA is only "nicked". On subsequent melting of the probe, the acceptor are donor molecules separate thereby generating a modified signal which can be detected.

This embodiment may be used in end-point detection, where the restriction enzyme is added on completion of the amplification. When it is to be used in the course of the reaction for example to monitor and/or quantify the amplification, it would be necessary to ensure that the restriction enzyme did not cut up the target molecule (which would generally not be resistant to restriction) prior to the commencement of the amplification. This may be achieved in various ways. The delivery of the restriction enzyme may be delayed until after at least the first round of amplification has taken place. This may be effected automatically, for example using the "hot start" techniques where polymerase enzyme is kept separate from the remainder of the reaction mixture, for example using a wax barrier, which melted to release the enzyme only after the desired temperature levels had been reached. Similar methods may be used in the assay of the invention to restrain the restriction enzyme until required in the process. Alternatively, other reaction suppression means can be employed, for example by including in the reaction mixture an antibody to the restriction enzyme which inhibits its activity but which is itself denatured at the desired temperature, or by using enzymes, in particular modified enzymes as are known in the art, such as TAQ Gold^{™} which require heating to a particular level before they are activated.

Suitable combinations of donor and acceptor molecules are known in the art. For example, the donor molecule may comprise a fluorescein dye and the acceptor molecule is a rhodamine dye.

Suitable conditions under which the amplification reaction can be carried out are well known in the art. The optimum conditions may be variable in each case depending upon the particular amplicon involved, the nature of the primers used and the enzymes employed. The optimum conditions may be determined in each case by the skilled person. Typical denaturation temperatures are of the order of 95°C, typical annealing temperatures are of the order of 55°C and extension temperatures are of the order of 72°C.

The use of oligonucleotide probes in the methods described aboveform a further aspect of the invention. In particular, these probes will comprise a donor molecule and an acceptor molecule and a site for a restriction enzyme which cuts at a specific double stranded DNA sequence located intermediate said donor and acceptor molecule.

The probes may include other features outlined above. For example, the probe may be in the form of a molecular beacon so that it has complementary sequences in the 5' region and the 3' region which bind together.

Suitable donor molecules include fluorescein dye such as fluroescein and the acceptor molecule may be a rhodamine dye or another dye such as Cy5 but any other dye combination which is capable of undergoing FRET interactions can be used.

A further aspect of the invention comprises a kit for carrying out a method as described above, said kit comprising a probe as described above and a restriction enzyme which is able to cut said probe intermediate the acceptor and donor molecules. The kit further comprises one modified nucleotide which is able to generate a nucleotide chain which is resistant to restriction enzymes, for instance methylated nucleotides.

The invention will now be particularly described by way of example with reference to the accompanying diagrammatic drawings in which:

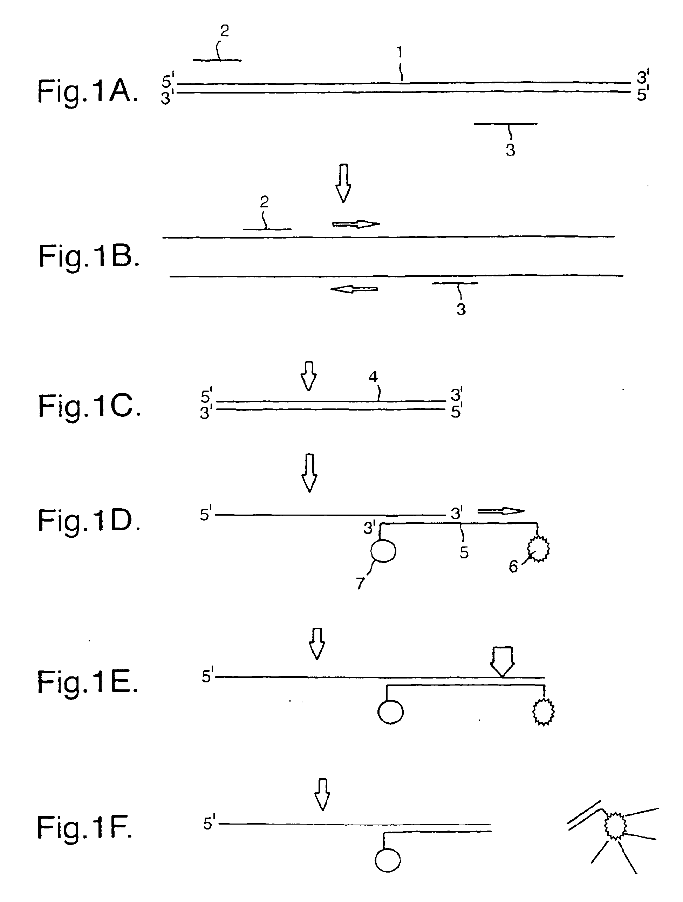

Figure 1 shows diagrammatically stages in reaction which forms a preferred embodiment of the invention;
Figure 2 illustrates method not falling within the scope of the invention;
Figure 3 illustrates yet a further method not falling within the scope of the invention;
Figure 4 is a graph showing fluorescence from a donar molecule vs time in a PCR reaction in accordance with the invention;
Figure 5 is a graph of the ratio of fluorescence of donar/fluorescence of acceptor in an end point PCR assay of the invention, where (A) and (B) are positive reactions and (c) represents a negative control;
Figure 6 is a graph of the ratio of fluorescence of acceptor/fluorescence of donar in an end point PCR assay of the invention, where (A) and (B) are positive reactions and (c) represents a negative control;
Figure 7 is a graph showing donor fluorescence with time an end point PCR assay of the invention, where (A) and (B) are positive reactions and (c) represents a negative control;
Figure 8 is a graph illustrating changes in fluorescence of donor (A) and acceptor (B) against time in the presence of a restriction enzyme which cuts between these; and
Figure 9 is a graph illustrating that cumulatively, the fluorescence increase in the course of the reaction giving rise to Figure 8.

In the illustrated amplification reaction, a DNA molecule (1) is first rendered single stranded (1B). A pair of amplification primers (2), (3) bind as forward and reverse primers in an amplification reaction as is well known. The result is an amplicon product (4) containing only the target sequence. When this product is melted during the subsequent phase of the amplification, probe (5) comprising a donor molecule (6) and an acceptor molecule (7) binds at the 3' end of the target sequence (1D). In the presence of a DNA polymerase, the target sequence will extend along the overhanging region of the probe, creating a double stranded site for a restriction enzyme indicated by the arrow.

The restriction enzyme is then able to cut the extended chain, liberating the acceptor molecule (7) from the donor molecule (6), thereby generating a signal (1F).

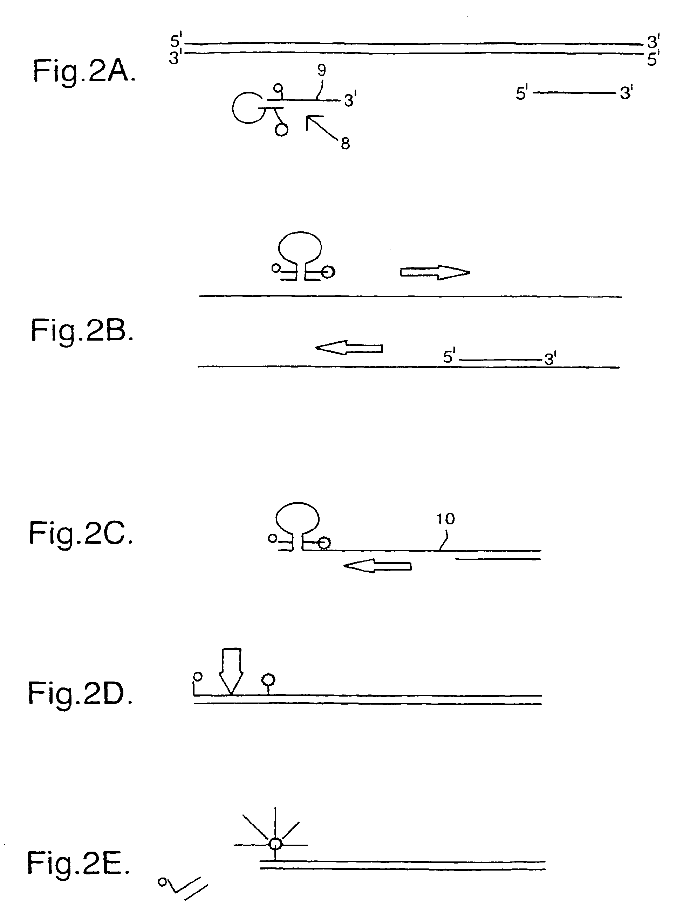

In the method of Figure 2, the probe (8) takes the form of a molecular beacon which is fused at the 5' end of an amplification primer (9). The probe-primer complex binds at one end of the target sequence when present and when melted into single-stranded form. Extension of the primer in the first cycle yields an amplicon strand (10) with the molecular beacon structure attached at the 5' end thereof. During the subsequent amplification cycle, the opposite amplification primer will bind at the 3' end of the amplicon strand (10), Extension of this strand will open the molecular beacon structure and also render it double stranded along its length. The thus formed double stranded site for the restriction enzyme may then be cut by that enzyme (2D), thereby still futher separating the donor molecule from the acceptor molecule (2E), and so producing an enhanced fluorescent signal.

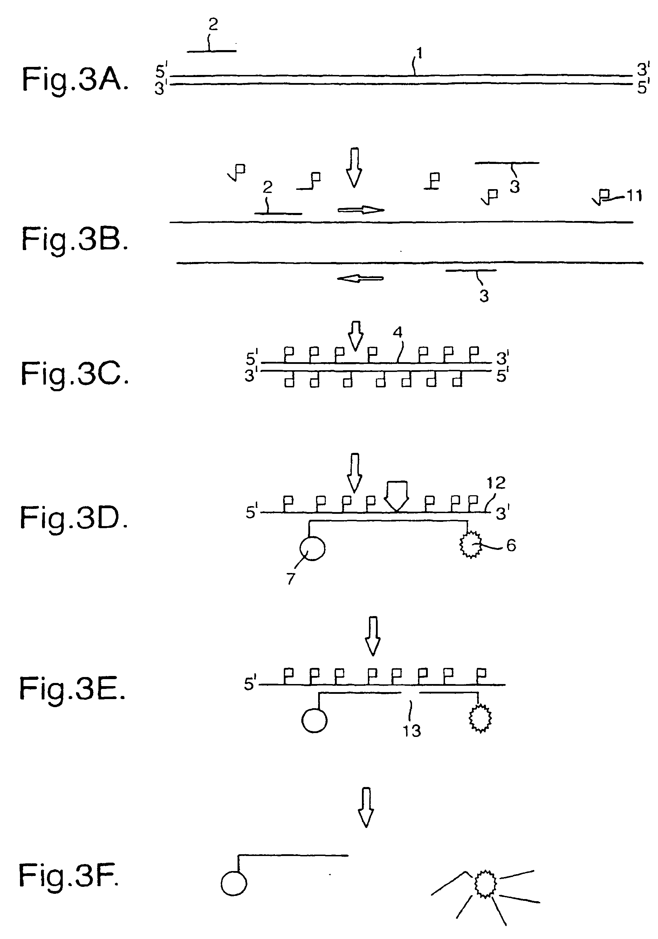

In the method illustrated in Figure 3, the amplification reaction is effected using modified nucleotides (11) such as methylated nucleotides. As a result, the amplicon strands (12)are resistant to restriction enzyme although the enzyme will recognise its restriction site (indicated by shaded arrow - 3D) when in double stranded form. When the amplicon strand (12) is hybridised to the probe which does not contain modified nucleotides, the result will be a "nick" (13) in the probe whilst the amplicon strand will remain intact. On subsequent melting (3F), the donor and acceptor molecules will separate allowing the donor molecule to generate an enhanced signal which may be detected.

Cutting with a restriction enzyme provides a rapid and effective means of separating the acceptor from donor molecule thus improving the speed of detection. Furthermore, by ensuring rapid and complete separation of acceptor from donor molecule, the signal to noise ratio is increased as compared to that obtainable using conventional molecular beacon technology.

The following Example is given by way of illustration.

### Demonstration 1

### Test Amplification Reactions

A 104 base pair amplicon of the anticoagulase gene of *Yersinia pestis* was cloned into pBluescript SK vector (Stratagene) to form pYP100ML phagemid construct. pYP100ML was amplified using forward primer YPPA155 of sequence:
dATGACGCAGAAACAGGAAGAAAGATCAGCC (SEQ ID NO 1)
and
reverse primer YPP229R of sequence:
dGGTCAGAAATGAGTATGGATCCCAGGATAT (SEQ ID NO 2)

PCR reaction conditions were as follows:
Primers 1µM (final) each
dNTPs 200µM (final) each
TAQ 0.025U/µl final concentration
buffer: 250ng/µl Bovine serum albumin, 500mM Tris, pH 8.3, 20mM magnesium

The mixture was then subjected to 30 cycles of the following conditions:

| Temperature °C | Time secs | Temperature Transition °C/sec | Fluoresence Monitoring |
|---|---|---|---|
| 95 Secondary 85 (1°C step per cycle) | 1 | 10 | |
| 55 | 1 | 10 | |
| 74 | 1 | 10 | Single (once per cycle) |

Amplification took under 6 minutes.

### Demonstration 2

A probe (YPPAMP), labelled at the 5' end with fluorescein as the donor and 10Bp downstream of that is the acceptor Cy5 label was prepared. The sequence of the probe is
5'T/CGAT/CCAGGXCAGAAATGAGTATGGATCCCAGGATAT 3, (SEQ ID NO 3)
where X represents the position of Cy5.

This probe should be able to act as the reverse primer in the PCR reaction detailed above. In between these two fluorescent molecules is a *Taq 1* restriction site (TCGA), marked with a "/" in the sequence. The 3' end has not been phosphorylated and.so it can be extended in the PCR reaction.

*Taq 1* endonuclease (Sigma), a type II enzyme, cleaves the double stranded DNA at this specific region. In doing so, it would separate the fluorescein donor from the Cy5 acceptor and this forms the basis of detection. The probe was designed so that the extra nucleotides were included at the 5' end, and the resulting pYP100ML product would be unchanged. The amplicon in this case contains no *Taq 1* sites.

The donor and acceptor molecules are positioned in sufficiently close proximity on the probe for FRET to occur. However, once the *Taq 1* enzyme cleaves the amplicon, the donar is released and FRET no longer occurs.

The YPPAMP probe was then utilised in a PCR reaction which had been optimised. The YPPAMP probe at 2 µM was included in a pYP100ML amplification using a Perkin Elmer 9700. The program was 30 cycles of:

| Temp °C | Time Seconds/minute |
|---|---|
| 96 | (30secs) |
| (50) | (30secs) |
| (72) | (4mins) |

The products were analysed by agarose gel electrophoresis. The product band was shown to be larger than the 104Bp pYP100ML native fragment using agarose gel electrophoresis suggesting that the probe had successfully amplified the target.

### Example 1

Thereafter, an amplification reaction as described in Demonstration 1 above was repeated with the YPPAMP probe replacing the YPP229R reverse primer.

After amplification, restriction enzyme *Taq 1* was added to PCR product along with dNTP's and palette buffer black (Sigma). Palette buffer black contains NaCl, that is required for Taq 1 endonuclease activity. The reaction was run on 1 cycle of:

| Temp °C | Time Seconds/minute |
|---|---|
| 65 | 60mins |

This program activates the cleaving activities of the endonuclease cutting the end of the fragment away. A negative control was run without enzyme and the fragments were analysed using gel electrophoresis. The banding pattern obtained indicated that the fragment had been cut by the restriction enzyme.

Both the enzyme cut and control samples were analysed using the fluorimeter. The PCR product was diluted so that the probe concentration in the reaction was 0.1µM. At higher concentration the fluorescence signal was too intense for the LightCycler^{™} detectors. The fluorescein fluorescence signal was higher than Cy5 in the test samples suggesting that the fluorescein was released and Cy5 was not close enough to quench its fluorescent energy.

The control samples had a high CY5 fluorescent reading suggesting that FRET was still occurring. The results indicated that the probe amplified and the endonuclease was effective in removing the end of the fragment.

### Example 2

The YPPAMP probe described above and *Taq 1* endonuclease were included in an amplification reaction using the LightCycler^{™}. Program below, 40 cycles of:

| Temperature °C | Time Secs | Temperature Transition °C/second | Monitoring |
|---|---|---|---|
| 95 | 0 | 10 | |
| 55 | 0 | 10 | |
| 74 | 20 | 10 | single |

The time for extension is increased from the usual pYP100ML amplification so the enzyme has time to cut the fragment.

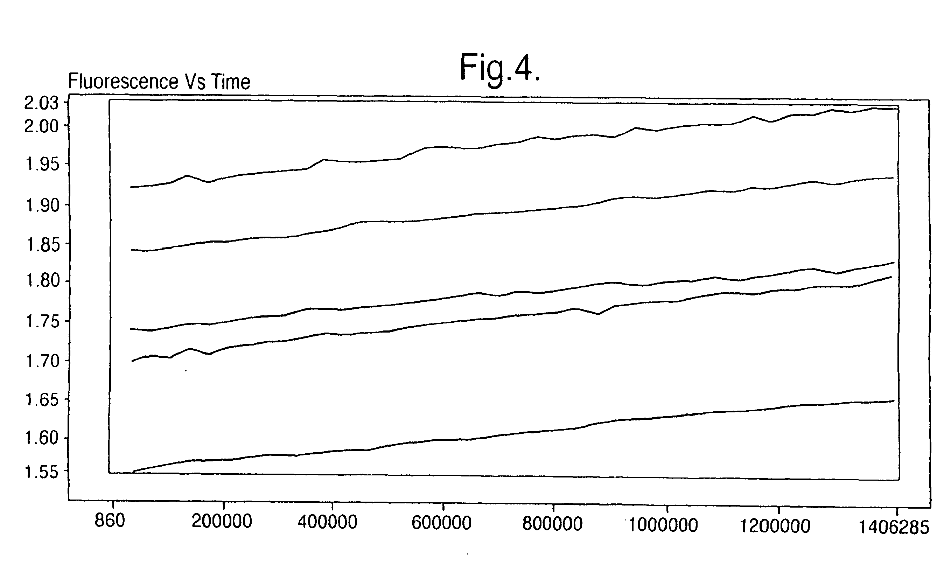

A fluorescence reading was taken once in every cycle and the results are shown in Figure 4. As the product amplifies the fluorescein fluorescence increased suggesting that the fluorescent molecules are being separated.

### Example 3

In order to improve the signal, the YPPAMP probe at 0.5µM was used in two similar amplification reactions to that described in Example 2 without *Taq 1*. Again a negative control was run in the absence of DNA. The program was the usual amplification program for pYP100ML, 30 cycles of:

| Temperature °C | Time Secs | Temperature Transition °C/second | Fluorescence Monitoring |
|---|---|---|---|
| 95 secondary 85 (1°C step per cycle) | 1 | 10 | |
| 55 | 1 | 10 | |
| 74 | 1 | 10 | Single |

After PCR amplification a digest was run with PCR product *Taq 1*, dNTP's and palette buffer black. The digest program was one cycle of:

| Temperature °C | Time Secs | Temperature Transition °C/second | Fluorescence Monitoring |
|---|---|---|---|
| 65 | 60 minutes | 20 | Continuous |

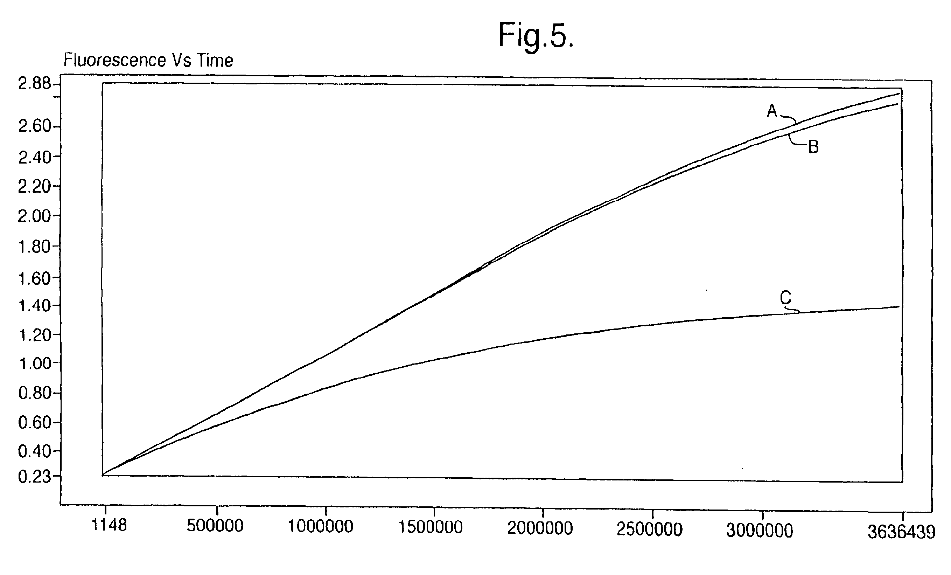

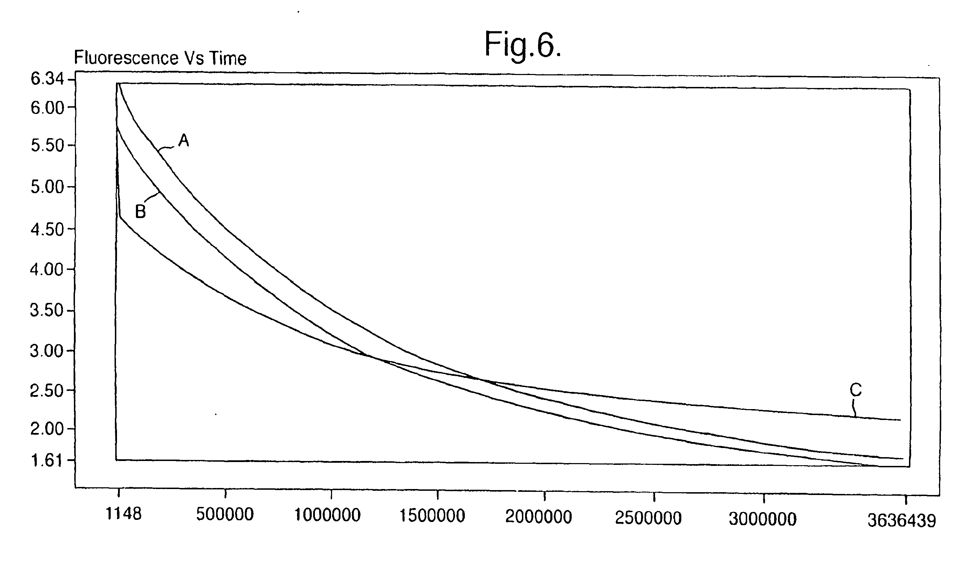

Figure 5 shows the results, expressed as a ratio of fluorescent signal of Fluorescein/Cy5 (F1/F2) vs time. As time increases the fluorescence increases in the two higher lines (A) and (B) which are positive samples. The results are shown again in Figure 6 in the form of a ratio of F1/1 vs time. A continuous rise in fluorescein fluorescence is clearly seen as *Taq 1* is digesting the end of the fragment, releasing the fluorescein. Possibly due to contamination the fluorescence of the negative sample (line C) increased slightly but the fluorescence was clearly much lower than the positive samples. pYP100ML amplicon is present in the surrounding air and as little as one amplicon from the air is needed in a reaction to amplity.

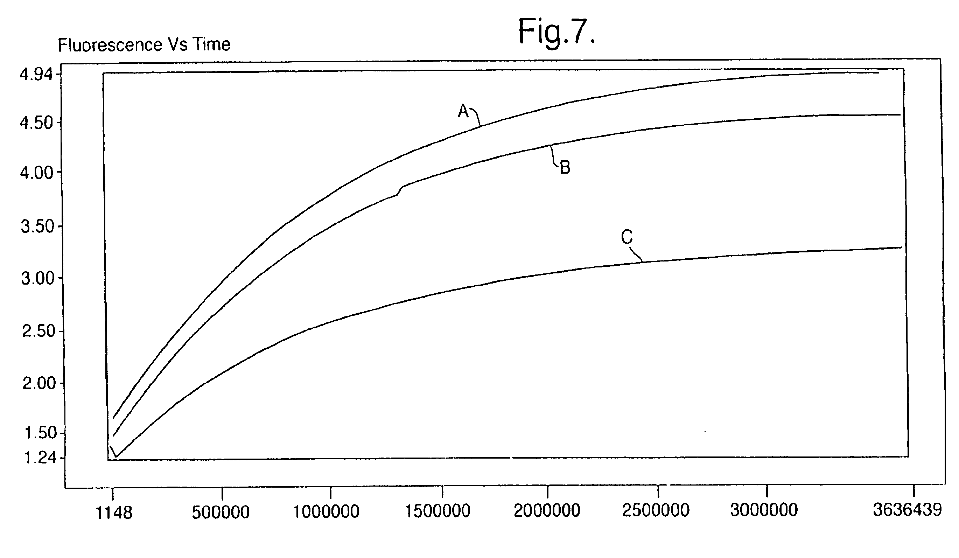

When expressed in the form of a graph of F2/1 vs time, (Figure 7), the decrease in fluorescence as FRET stops between fluorescein and Cy5 is apparent.

### Example 4

### Probe Digestion experiments

A sample of the YPPAMP probe was digested by Taq 1 over a period of 60 minutes, under the conditions outlined in Example 3.

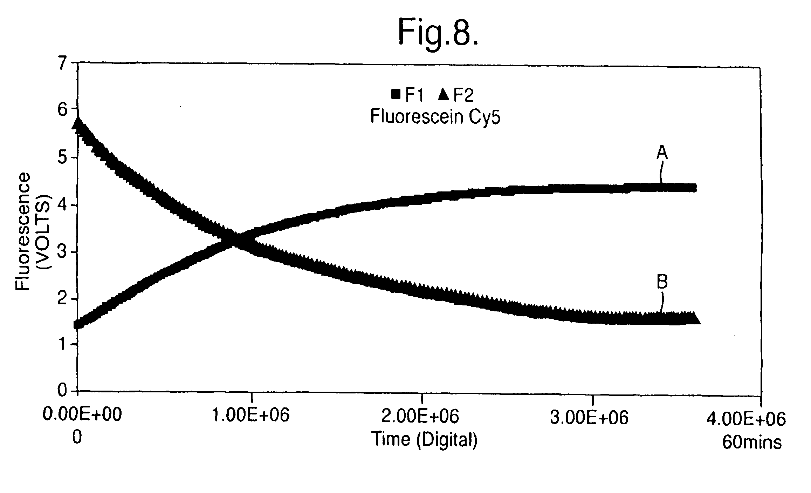

Figure 8 shows the fluorescence at the donor and acceptor wavelengths as the probe is cut by the restriction enzyme over time.

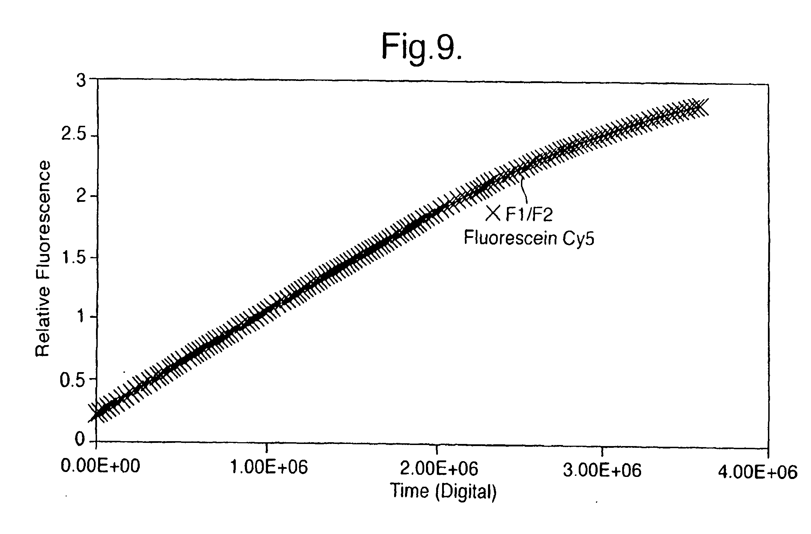

Initially, the probe is intact with a high Cy5 signal (B) and a low fluorescein signal. As the probe is cut, the fluorescein signal (A) rises as it is released from the probe and a fall in the Cy5 signal occurs as the molecules are no longer in close proximity for FRET to occur. Figure 9 illustrates the relative fluorescence over time F1/F2 (fluorescein/Cy5). This shows a continuous rise overall, as a result of the cutting of the probe.

## Claims

1. A method for detecting nucleic acid amplification in a sample, comprising performing nucleic acid amplification on a target polynucleotide in the presence of (a) a nucleic acid polymerase, (b) a primer capable of hybridising to said target polynucleotide, and (c) an oligonucleotide probe which is capable of hybridising to said target polynucleotide and which comprises a fluorescence donor and a fluorescence acceptor molecule spaced along the length at a distance at which fluorescence from the donor is reduced by the acceptor, and a single stranded sequence recognised by a restriction enzyme which cuts at said sequence when in double stranded form, said sequence being located intermediate said donor and said acceptor molecule; applying to the amplification product said restriction enzyme such that double stranded amplification product is cleaved so as to liberate acceptor molecule from donor molecule, and detecting a fluorescent signal from the sample, wherein said oligonucleotide probe is capable of hybridising to said target polynucleotide in the region of the 3' end of said target polynucleotide such that the 3' end of the polynucleotide can extend during amplification to form a complementary region of the probe.

2. A method according to claim 1 wherein said restriction enzyme is present throughout the amplification reaction and the progress of the amplification reaction is monitored by measuring the increase in the signal from the donor molecule.

3. A method according to either of the preceding claims wherein said restriction enzyme is Taq1 or PspG1.

4. A method according to any one of claims 1 to 3 wherein the nucleic acid polymerase is a thermostable polymerase.

5. A method according to any one of claims 1 to 4 wherein the probe has complementary sequences in the 5' and the 3' region such that prior to binding to the target polynucleotide sequence, the 5' region and the 3' region bind together.

6. A method according to any of the preceding claims wherein said donor molecule comprises a fluorescein dye.

7. A method according to any one of the preceding claims wherein said acceptor molecule is a rhodamine dye or Cy5.

8. A method according to any one of claims 1 to 7 wherein the donor molecule is attached to a nucleotide in the region of the 3' end of the probe and the acceptor molecule is attached in the region of the 5' end of the probe.

9. A method according to any one of claims 1 to 7 wherein the donor molecule is attached to a nucleotide in the region of the 5' end of the probe and the acceptor molecule is attached to a nucleotide in the region of the 3' end of the probe.

10. A method according to any one of claims 1 to 9 wherein the amplification reaction is effected using modified nucleotides which are resistant to cutting by restriction enzymes.

11. A method according to claim 10 wherein the modified nucleotides are methylated nucleotides.

12. A method according to claim 10 or claim 11 wherein the restriction enzyme is inhibited from acting until the amplification reaction has proceeded so that at least some amplicon product exists.

13. A method according to claim 12 where restriction enzyme is kept separate from the remainder of the reaction mixture, until some amplicon product is obtained.

14. A method according to claim 13 wherein the restrictions enzyme is restrained by a meltable barrier.

15. A method according to claim 12 wherein the restriction enzyme is inhibited by the presence of an antibody.

16. A method according to claim 12 wherein the restriction enzyme is an enzyme which become active only on exposure to particular temperatures.

17. A method according to any one of claims 1 to 16 wherein the amplification reaction is effected by the polymerase chain reaction (PCR).

18. A method according to any one of claims 1 to 17 wherein the probe is attached at the 5' end of an amplification primer.

19. The use of an oligonucleotide probe which comprises a donor molecule and an acceptor molecule and a site for a restriction enzyme which cuts at a specific double stranded DNA sequence located intermediate said donor and acceptor molecule, in a method according to any one of the preceding claims.

20. The use according to claim 19 wherein said donor molecule comprises a fluorescein dye and said acceptor molecule is a rhodamine dye or Cy5.

21. The use according to claim 19 wherein in the probe the said donor molecule is separated from said acceptor molecule by at least 15 nucleotides.

22. The use according to claims 19 or claim 20 wherein the donor molecule is attached to a nucleotide in the region of the 3' end of the probe and the acceptor molecule is attached to a nucleotide in the region of the 5' end of the probe.

23. The use according to any one of claims 19 to 21 wherein the donor molecule is attached to a nucleotide in the region of the 5' end of the probe and the acceptor molecule is attached to a nucleotide in the region of the 3' end of the probe.

24. The use according to any one of claims 19 to 22 wherein said oligonucleotide probe is attached at the 5' end of an amplification primer.

25. A kit for carrying out a method according to any one of claims 1 to 18, said kit comprising an oligonucleotide probe which is capable of hybridising to a target polynucleotide and which comprises a fluorescence donor molecule a fluorescence acceptor moleculs and a single stranded sequence recognised by a restriction enzyme which cuts at said sequence when in double stranded form, said sequence being located intermediate said donor and said acceptor molecule, a restriction enzyme which is able to cut said probe intermediate the acceptor and donor molecules and a modified nucleotide which is able to generate a nucleotide chain which is resistant to restriction enzymes.

26. A kit according to claim 25 wherein the modified nucleotide comprises a methylated nucleotide

## Patentansprüche

1. Verfahren zur Erfassung einer Nucleinsäure-Amplifizierung in einer Probe, das umfasst:
Durchführen einer Nucleinsäure-Amplifizierung an einem Target-Polynucleotid in Gegenwart
(a) einer Nucleinsäurepolymerase,
(b) eines Primers, der befähigt ist, mit dem Target-Polynucleotid zu hybridisieren, und
(c) einer Oligonucleotid-Sonde, die befähigt ist, mit dem Target-Polynucleotid zu hybridisieren, und ein Fluoreszenz-Donor-Molekül und ein Fluoreszenz-Akzeptor-Molekül, die über seine Länge in einem Abstand voneinander beabstandet sind, bei dem die Fluoreszenz vom Donor durch den Akzeptor verringert ist, und eine einzelsträngige Sequenz aufweist, die von einem Restriktionsenzym erkannt wird, das diese Sequenz schneidet, wenn sie in doppelsträngiger Form vorliegt, wobei diese Sequenz zwischen dem Donor- und dem Akzeptor-Molekül angeordnet ist,
Anwenden des Restriktionsenzyms auf das Amplifizierungsprodukt, so dass das doppelsträngige Amplifizierungsprodukt so gespalten wird, dass das Akzeptor-Molekül vom Donor-Molekül freigesetzt wird,
und
Erfassen eines Fluoreszenzsignals von der Probe,
wobei die Oligonucleotid-Sonde befähigt ist, mit dem Target-Polynucleotid im Bereich seines 3'-Endes so zu hybridisieren, dass das 3'-Ende des Polynucleotids während der Amplifizierung unter Bildung eines komplementären Bereichs der Sonde verlängert werden kann.

2. Verfahren nach Anspruch 1, bei dem das Restriktionsenzym während der gesamten Amplifizierungsreaktion vorliegt, und das Fortschreiten der Amplifizierungsreaktion durch Messung der Zunahme des Signals vom Donor-Molekül überwacht wird.

3. Verfahren nach einem der vorhergehenden Ansprüche, bei dem das Restriktionsenzym Taq1 oder PspG1 ist.

4. Verfahren nach einem der Ansprüche 1 bis 3, bei dem die Nucleinsäurepolymerase eine thermostabile Polymerase ist.

5. Verfahren nach einem der Ansprüche 1 bis 4, bei dem die Sonde solche komplementären Sequenzen im 5'- und 3'-Bereich aufweist, dass der 5'-Bereich und der 3'-Bereich vor der Bindung an die Target-Polynucleotidsequenz eine Bindung miteinander eingehen.

6. Verfahren nach einem der vorhergehenden Ansprüche, bei dem das Donor-Molekül ein Fluorescein-Farbstoff ist.

7. Verfahren nach einem der vorhergehenden Ansprüche, bei dem das Akzeptor-Molekül ein Rhodamin-Farbstoff oder Cy5 ist.

8. Verfahren nach einem der Ansprüche 1 bis 7, bei dem das Donor-Molekül an einem Nucleotid im Bereich des 3'-Endes der Sonde angebracht ist und das Akzeptor-Molekül im Bereich des 5'-Endes der Sonde angebracht ist.

9. Verfahren nach einem der Ansprüche 1 bis 7, bei dem das Donor-Molekül an einem Nucleotid im Bereich des 5'-Endes der Sonde angebracht ist und das Akzeptor-Molekül an einem Nucleotid im Bereich des 3'-Endes der Sonde angebracht ist.

10. Verfahren nach einem der Ansprüche 1 bis 9, bei dem die Amplifizierungsreaktion unter Verwendung von modifizierten Nucleotiden durchgeführt wird, die gegen Schneiden durch Restriktionsenzyme resistent sind.

11. Verfahren nach Anspruch 10, bei dem die modifizierten Nucleotide methylierte Nucleotide sind.

12. Verfahren nach Anspruch 10 oder 11, bei dem das Restriktionsenzym an seiner Wirkung gehindert wird, bis die Amplifizierungsreaktion so weit fortgeschritten ist, dass mindestens etwas Amplicon-Produkt vorliegt.

13. Verfahren nach Anspruch 12, bei dem das Restriktionsenzym vom Rest des Reaktionsgemischs getrennt gehalten wird, bis etwas Amplicon-Produkt erhalten ist.

14. Verfahren nach Anspruch 13, bei dem das Restriktionsenzym durch eine schmelzbare Barriere zurückgehalten wird.

15. Verfahren nach Anspruch 12, bei dem das Restriktionsenzym durch das Vorliegen eines Antikörpers inhibiert wird:

16. Verfahren nach Anspruch 12, bei dem das Restriktionsenzym ein Enzym ist, das lediglich dann aktiv wird, wenn es bestimmten Temperaturen ausgesetzt wird.

17. Verfahren nach einem der Ansprüche 1 bis 16, bei dem die Amplifizierungsreaktion durch Polymerase-Kettenreaktion (PCR) vorgenommen wird.

18. Verfahren nach einem der Ansprüche 1 bis 17, bei dem die Sonde am 5'-Ende eines Amplifizierungsprimers angebracht ist.

19. Verwendung einer Oligonucleotid-Sonde, die ein Donor-Molekül und ein Akzeptor-Molekül sowie eine Stelle für ein Restriktionsenzym aufweist, das die doppelsträngige DNA-Sequenz, die sich zwischen dem Donor-Molekül und dem Akzeptor-Molekül befindet, an einer spezifischen Stelle schneidet, in einem Verfahren nach einem der vorhergehenden Ansprüche.

20. Verwendung nach Anspruch 19, wobei das Donor-Molekül ein Fluorescein-Farbstoff und das Akzeptor-Molekül ein Rhodamin-Farbstoff oder Cy5 ist.

21. Verwendung nach Anspruch 19, wobei in der Sonde das Donor-Molekül durch mindestens 15 Nucleotide vom Akzeptor-Molekül getrennt ist.

22. Verwendung nach Anspruch 19 oder 20, wobei das Donor-Molekül an einem Nucleotid im Bereich des 3'-Endes der Sonde angebracht ist und das Akzeptor-Molekül im Bereich des 5'-Endes der Sonde angebracht ist.

23. Verwendung nach einem der Ansprüche 19 bis 21, wobei das Donor-Molekül an einem Nucleotid im Bereich des 5'-Endes der Sonde angebracht ist und das Akzeptor-Molekül an einem Nucleotid im Bereich des 3'-Endes der Sonde angebracht ist.

24. Verwendung nach einem der Ansprüche 19 bis 22, wobei die Oligonucleotid-Sonde am 5'-Ende eines Amplifizierungsprimers angebracht ist.

25. Kit zur Durchführung eines Verfahrens nach einem der Ansprüche 1 bis 18, der umfasst:
eine Oligonucleotid-Sonde, die befähigt ist, mit einem Target-Polynucleotid zu hybridisieren, und ein Fluoreszenz-Donor-Molekül, ein Fluorezenz-Akzeptor-Molekül und eine einzelsträngige Sequenz aufweist, die durch ein Restriktionsenzym erkannt wird, das diese Sequenz schneidet,
wenn sie in doppelsträngiger Form vorliegt, wobei diese Sequenz zwischen dem Donor- und dem Akzeptor-Molekül angeordnet ist,
ein Restriktionsenzym, das befähigt ist, die Sonde zwischen dem Akzeptor-Molekül und dem Donor-Molekül zu schneiden, und
ein modifiziertes Nucleotid, das befähigt ist, eine Nucleotidkette zu erzeugen, die gegen Restriktionsenzyme resistent ist.

26. Kit nach Anspruch 25, bei dem das modifizierte Nucleotid ein methyliertes Nucleotid ist.

## Revendications

1. Procédé pour détecter une amplification d'acide nucléique dans un échantillon, comprenant la réalisation d'une amplification d'acide nucléique sur un polynucléotide cible en présence (a) d'une acide nucléique polymérase, (b) d'une amorce capable de s'hybrider avec ledit polynucléotide cible, et (c) d'une sonde oligonucléotidique qui est capable de s'hybrider avec ledit polynucléotide cible et qui comprend une molécule donneuse de fluorescence et une molécule acceptrice de fluorescence espacées sur la longueur à une distance à laquelle la fluorescence provenant du donneur est réduite par l'accepteur, et une séquence simple brin reconnue par une enzyme de restriction qui coupe au niveau de ladite séquence lorsqu'elle est sous une forme double brin, ladite séquence étant située entre ladite molécule donneuse et ladite molécule acceptrice ; l'application de ladite enzyme de restriction au produit d'amplification de sorte que le produit d'amplification double brin soit clivé de manière à libérer la molécule acceptrice de la molécule donneuse, et la détection d'un signal fluorescent provenant de l'échantillon, où ladite sonde oligonucléotidique est capable de s'hybrider avec ledit polynucléotide cible dans la région de l'extrémité 3' dudit polynucléotide cible de sorte que l'extrémité 3' du polynucléotide puisse s'étendre au cours de l'amplification pour former une région complémentaire de la sonde.

2. Procédé selon la revendication 1, dans lequel ladite enzyme de restriction est présente durant la réaction d'amplification et l'évolution de la réaction d'amplification est contrôlée en mesurant l'augmentation du signal provenant de la molécule donneuse.

3. Procédé selon l'une des revendications précédentes, dans lequel ladite enzyme de restriction est Taq1 ou PspG1.

4. Procédé selon l'une quelconque des revendications 1 à 3, dans lequel l'acide nucléique polymérase est une polymérase thermostable.

5. Procédé selon l'une quelconque des revendications 1 à 4, dans lequel la sonde a des séquences complémentaires dans la région 5' et la région 3' de sorte qu'avant la liaison à la séquence de polynucléotide cible, la région 5' et la région 3' se lient ensemble.

6. Procédé selon l'une des revendications précédentes, dans lequel ladite molécule donneuse comprend un colorant de fluorescéine.

7. Procédé selon l'une quelconque des revendications précédentes, dans lequel ladite molécule acceptrice est un colorant de rhodamine ou Cy5.

8. Procédé selon l'une quelconque des revendications 1 à 7 dans lequel la molécule donneuse est fixée à un nucléotide dans la région de l'extrémité 3' de la sonde et la molécule acceptrice est fixée dans la région de l'extrémité 5' de la sonde.

9. Procédé selon l'une quelconque des revendications 1 à 7, dans lequel la molécule donneuse est fixée à un nucléotide dans la région de l'extrémité 5' de la sonde et la molécule acceptrice est fixée à un nucléotide dans la région de l'extrémité 3' de la sonde.

10. Procédé selon l'une quelconque des revendications 1 à 9, dans lequel la réaction d'amplification est effectuée en utilisant des nucléotides modifiés qui sont résistants à la coupure par des enzymes de restriction

11. Procédé selon la revendication 10, dans lequel les nucléotides modifiés sont des nucléotides méthylés.

12. Procédé selon la revendication 10 ou 11, dans lequel l'enzyme de restriction est empêchée d'agir jusqu'au déroulement de la réaction d'amplification de sorte qu'au moins une certaine quantité de produit d'amplicon existe.

13. Procédé selon la revendication 12 dans lequel l'enzyme de restriction est maintenue séparée du reste du mélange réactionnel; jusqu'à l'obtention d'une certaine quantité de produit d'amplicon.

14. Procédé selon la revendication 13, dans lequel l'enzyme de restriction est retenue par une barrière pouvant fondre.

15. Procédé selon la revendication 12, dans lequel l'enzyme de restriction est inhibée par la présence d'un anticorps.

16. Procédé selon la revendication 12, dans lequel l'enzyme de restriction est une enzyme qui ne devient active que lors d'une exposition à des températures particulières.

17. Procédé selon l'une quelconque des revendications 1 à 16, dans lequel la réaction d'amplification est effectuée par la réaction en chaîne par polymérase (PCR).

18. Procédé selon l'une quelconque des revendications 1 à 17, dans lequel la sonde est fixée au niveau de l'extrémité 5' d'une amorce d'amplification.

19. Utilisation d'une sonde oligonucléotidique qui comprend une molécule donneuse et une molécule acceptrice et un site pour une enzyme de restriction qui coupe au niveau d'une séquence d'ADN double brin spécifique située entre ladite molécule donneuse et ladite molécule acceptrice, dans un procédé selon l'une quelconque des revendications précédentes.

20. Utilisation selon la revendication 19, dans laquelle ladite molécule donneuse comprend un colorant de fluorescéine et ladite molécule acceptrice est un colorant de rhodamine ou Cy5.

21. Utilisation selon la revendication 19, dans laquelle, dans la sonde, ladite molécule donneuse est séparée de ladite molécule acceptrice par au moins 15 nucléotides.

22. Utilisation selon la revendication 19 ou 20, dans laquelle la molécule donneuse est fixée à un nucléotide dans la région de l'extrémité 3' de la sonde et la molécule acceptrice est fixée à un nucléotide dans la région de l'extrémité 5' de la sonde.

23. Utilisation selon l'une quelconque des revendications 19 à 21, dans laquelle la molécule donneuse est fixée à un nucléotide dans la région de l'extrémité 5' de la sonde et la molécule acceptrice est fixée à un nucléotide dans la région de l'extrémité 3' de la sonde.

24. Utilisation selon l'une quelconque des revendications 19 à 22, dans laquelle ladite sonde oligonucléotidique est fixée au niveau de l'extrémité 5' d'une amorce d'amplification.

25. Kit pour mettre en oeuvre un procédé selon l'une quelconque des revendications 1 à 18, ledit kit comprenant une sonde oligonucléotidique qui est capable de s'hybrider avec un polynucléotide cible et qui comprend une molécule donneuse de fluorescence, une molécule acceptrice de fluorescence et une séquence simple brin reconnue par une enzyme de restriction qui coupe au niveau de ladite séquence lorsqu'elle est sous une forme double brin, ladite séquence étant située entre ladite molécule donneuse et ladite molécule acceptrice, une enzyme de restriction qui est capable de couper ladite sonde entre les molécules acceptrice et donneuse et un nucléotide modifié qui est capable de générer une chaîne nucléotidique qui est résistante aux enzymes de restriction.

26. Kit selon la revendication 25, dans lequel le nucléotide modifié comprend un nucléotide méthylé.
